# FASCICULE DE BREVET EUROPEEN

(11) **EP 0 441 729 B1**
(45) Date de publication et mention de la délivrance du brevet: **12.01.1994**
(21) Numéro de dépôt: 91450001.2
(22) Date de dépôt: 08.02.1991
(51) Int. Cl.: A61B 17/60

(54) **Dispositif de fixation orientable de tiges d'ostéosynthèse rachidienne**
Schwenkbare Befestigungsvorrichtung für Rückgratostheosynthesestangen
Swivelling fastening device for spinal osteosynthesis rods

(30) Priorité: 08.02.1990 FR 9001634; 19.03.1990 FR 9003694
(43) Date de publication de la demande: 14.08.1991
(73) Titulaire: STRYKER CORPORATION (a Michigan corporation), Kalamazoo, Michigan 49002 (US); LAPRESLE, Philippe, F-92200 Neuilly-sur-Seine (FR); MISSENARD, Gilles, F-75016 Paris (FR)
(72) Inventeur: Vignaud, Jean-Louis, F-33950 Le Piraillan - Lege - Cap Ferret (FR); Lapresle, Philippe, F-92200 Neuilly-sur-Seine (FR); Missenard, Gilles, F-75016 Paris (FR); Sacriste, Jean-François, F-33115 Le Pyla-sur-Mer (FR)
(74) Mandataire: Martin, Jean-Jacques

(56) Documents cités:
- EP-A- 0 077 159
- EP-A- 0 330 881
- EP-A- 0 348 272
- WO-A-89/00028
- DE-B- 2 834 891
- FR-A- 2 559 378
- FR-A- 2 624 720
- FR-A- 2 642 643
- US-A- 4 836 196

## Description

La présente invention a trait à une instrumentation rachidienne orientable pour fixation pédiculaire et vise à permettre d'adapter et de rétablir les courbures physiologiques du rachis.

Habituellement on implante des vis pédiculaires réunies par des tiges ou des plaques, la vis demeurant perpendiculaire à la tige ou à la plaque. Un exemple d'une telle fixation d'une tige est donné par le document **GB-A-2 173 104.**

Dans le document **WO-A-8900028** on décrit un dispositif permettant de donner une certaine angulation à la tige par rapport à la vis pédiculaire, toutefois, la tige doit être filetée et le système de blocage de la tige par écrous et contre-écrous engagés sur la tige filetée et tête de vis sphérique est complexe et peu pratique.

L'invention vise à remédier à ces divers inconvénients et à proposer un dispositif de fixation de tiges lisses permettant une orientation de celles-ci vis à vis de l'axe des vis pédiculaires.

A cet effet, l'invention a pour objet un dispositif de fixation de tiges d'ostéosynthèse rachidienne sur des vis pédiculaires du type comprenant une tête en forme de diapason définissant deux branches parallèles en regard délimitant entre elles un logement d'axe général perpendiculaire à l'axe de la vis, susceptible de recevoir une tige à fixer, laquelle est bloquée dans son logement par une vis de serrage engagée entre lesdites branches, caractérisé en ce qu'il comporte, en outre, enfilée sur la tige et interposée entre la vis de serrage et le fond dudit logement, une bague fendue dont les faces externes opposées dirigées vers ladite vis de serrage et ledit fond sont convexes et reçues dans des surfaces concaves complémentaires ménagées sur l'extrémité de la vis de serrage et le fond du logement de réception de la tige, ladite bague autorisant, avant serrage de la vis, un certain débattement angulaire de la tige par rapport à l'axe de la vis.

Un tel dispositif permet d'ajuster l'angle formé par la tige avec l'axe de la vis pédiculaire, en particulier dans le plan sagittal, et de bloquer la tige dans la position désirée grâce à la bague fendue qui se déforme et immobilise la tige sous la pression de la vis de serrage.

D'autres caractéristiques et avantages ressortiront de la description qui va suivre d'un mode de réalisation du dispositif de l'invention, description donnée à titre d'exemple uniquement et en regard des dessins annexés sur lesquels :
- Figure 1 est une vue en éclaté d'un dispositif conforme à l'invention ;
- Figure 2 représente le dispositif de la figure 1 assemblé, excepté le capuchon ;
- Figure 3 est une coupe suivant le plan sagittal d'une vis pédiculaire selon l'invention ;
- Figure 4 est une vue en perspective d'une bague fendue selon l'invention ;
- Figure 5 est une vue en élévation latérale de la bague de la figure 4 ;
- Figure 6 est une vue en coupe axiale d'une vis de serrage selon l'invention, et
- Figure 7 est une vue agrandie de l'extrémité de la tige de la figure 1.

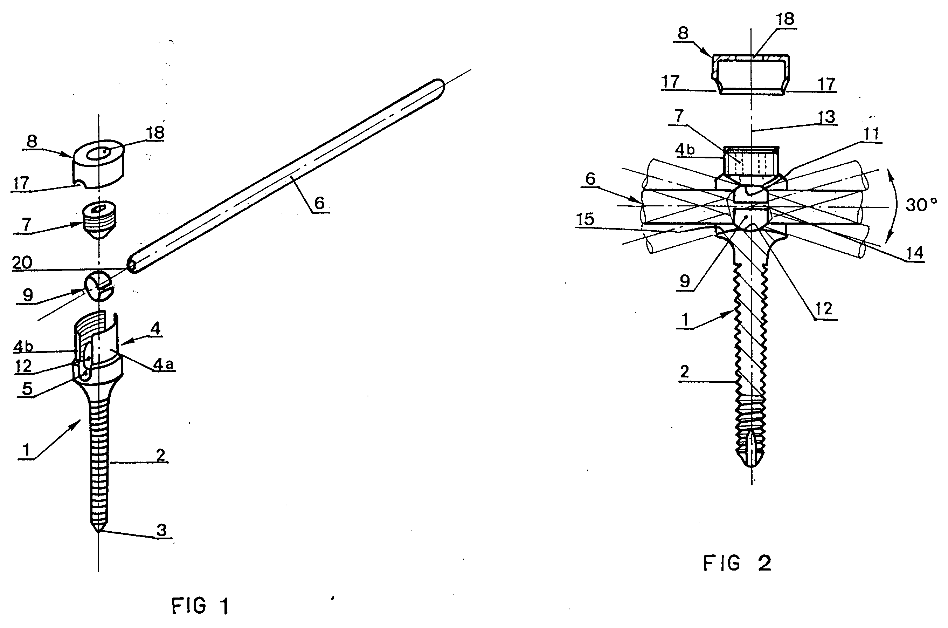

La figure 1 représente en 1 une vis pédiculaire de conception connue. Elle comporte une vis conique 2, une pointe ogivale 3, une tête cylindrique 4 percée de part en part et en forme de diapason. Plus précisément la tête 4 comporte deux branches paralléles en regard 4a,4b définissant entre elles une échancrure ou logement 5 d'axe général perpendiculaire à l'axe de la vis 2, susceptible de recevoir une tige ou élément de solidarisation 6 de forme générale cylindrique. Une telle structure est bien connue et est décrite par exemple dans le document GB-2.173.104.

Les faces internes en regard des branches 4a,4b sont cylindriques et taraudées de façon à recevoir une vis de serrage 7 de blocage de la tige 6 dans son logement 5 à la manière du dispositif de blocage à vis décrit dans EP-0.010.527.

La tête 4 reçoit un capuchon 8 coiffant les extrémités supérieures des branches 4a,4b.

Conformément à l'invention, le blocage de la tige 6 est assuré par l'intermédiaire d'une bague fendue 9 pouvant coulisser librement sur la tige 6, reçue dans le logement 5 et serrée par la vis de serrage 7.

La bague 9, l'extrémité de la vis de serrage 7 et la partie du logement 5 en contact avec la bague 9 sont conformées de manière particulière.

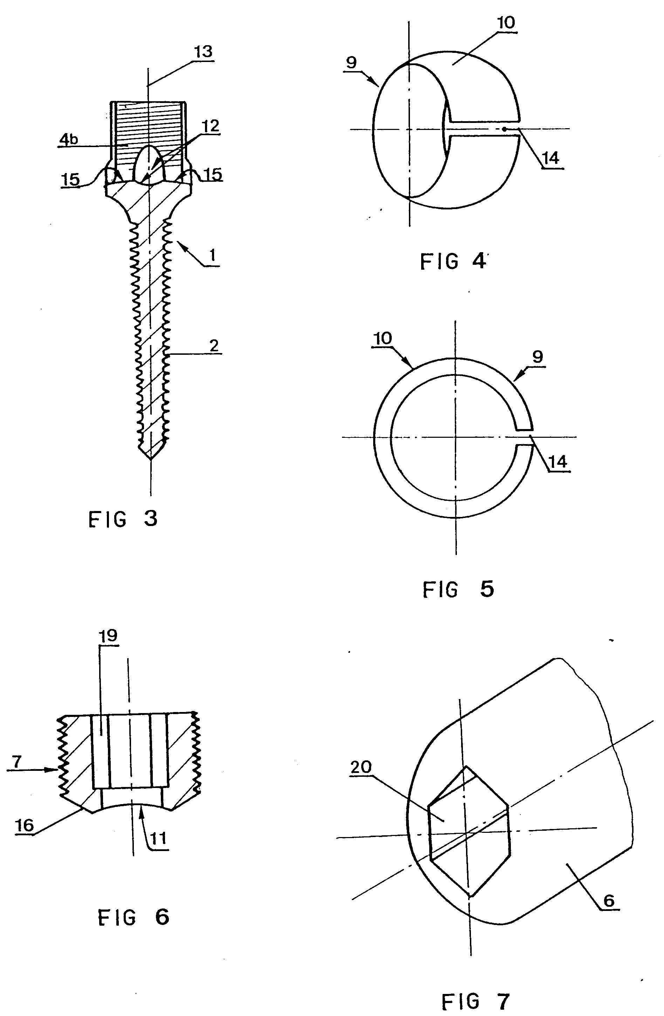

Dans le mode de réalisation représenté, la bague 9 comporte (figure 4) une surface externe 10 convexe sphérique coopérant, d'une part, avec une surface concave sphérique 11 ménagée (figure 6) à l'extrémité de la partie conique de la vis de serrage 7 et, d'autre part, avec une surface concave sphérique 12 ménagée (figure 3) dans le fond du logement 5 de réception entre les branches 4a,4b de la tige 6.

La bague 9 est agencée de façon que son plan soit sensiblement perpendiculaire (figure 2) au plan sagittal de la vis 1, lequel est défini par l'axe 13 de la vis et l'axe général du logement 5 recevant la tige 6.

La bague 9 est munie d'une fente droite 14 (figures 4 et 5) et présente une face interne cylindrique 15 très légèrement supérieure au diamètre de la tige 6.

La tige 6 sur laquelle est enfilée une bague 9 est insérée dans le logement 5 de la vis pédiculaire 1, après mise en place de cette dernière dans la partie osseuse appropriée. La bague 9 est engagée dans le logement 5,12 comme représenté en figure 2, puis la vis de serrage 7 est vissée dans l'espace entre les branches 4a,4b. Avant le serrage de la bague 9, le capuchon 8 est mis en place en étant simplement enfilé à cheval sur les deux branches 4a,4b, puis la tige 6 est convenablement orientée dans ledit plan sagittal (figure 2) et bloquée avec l'orientation désirée par compression de la bague fendue 9 par la vis de serrage 7. Le débattement angulaire de la tige 6 est par exemple de 15° autour de la position perpendiculaire à l'axe 13 de la vis 1 comme illustré par la figure 2.

Au cours de la modification de l'orientation de la tige 6, la bague 9 glisse sur les surfaces sphériques 11,12 entre lesquelles elle est emprisonnée.

Pour permettre le libre débattement angulaire de la tige 6 le fond du logement 5 au droit des deux entrées opposées est légèrement évidé en 15 (figures 2 et 3), la partie tronconique subsistante 16 de la pointe de la vis de serrage 7 (figure 6) assurant le libre débattement de la tige du côté vis de serrage.

Le capuchon 8 comporte (figure 2) deux échancrures opposées 17 pour le libre débattement de la tige 6 et un perçage central 18 pour le passage d'une clé, par exemple à six pans, de serrage de la vis de serrage 7 par l'intermédiaire d'une empreinte à six pans creuse 19 ménagée à la manière connue dans la face supérieure de ladite vis 7.

Le rôle du capuchon est essentiellement d'empêcher l'écartement des deux branches 4a,4b lors du serrage de la vis de serrage. Le capuchon 8 peut être supprimé si le matériau de la vis 1 ou le dimensionnement des branches 4a,4b, en particulier l'épaisseur, le permet.

Le serrage à fond de la vis de serrage 7 provoque le blocage par la bague 9 de la tige 6 dans la position choisie, la fente 14 étant de préférence placée latéralement comme illustré par la figure 2.

La tige est ainsi parfaitement bloquée à la fois en orientation et en rotation autour de son axe.

La tige 6 est par exemple un élément de solidarisation lisse muni à ses deux extrémités d'une empreinte 20 du type à six pans creux (figure 7) ou bien un élément constitué de torons cylindriques souples tressés.

La prévision des trous ou empreintes 20 permet l'introduction d'une clé de forme complémentaire en vue d'agir sur la dérotation de la colonne vertébrale.

En effet, en agissant sur les clés par rotation dans le même sens de la tige 6 sur son axe, on fait tourner cette dernière et, par conséquent, le rachis suivant son axe vertical.

Enfin, l'invention n'est évidemment pas limitée au mode de réalisation représenté et décrit ci-dessus mais en couvre au contraire toutes les variantes notamment en ce qui concerne la forme et l'agencement des surfaces convexes et concaves respectivement de la bague 9 et des surfaces 11 et 12 entre lesquelles elle est serrée et en ce qui concerne la vis de serrage 7 qui peut être vissée dans les branches en regard 4a,4b ou dans une pièce rapportée sur la tête 4 de la vis. De même les formes et dimensions de la bague 9 et de la fente 14 peuvent également varier sans sortir du cadre de l'invention dans la mesure où la fente joue le même rôle de coincement élastique de la bague par écrasement sur la tige à bloquer.

Les extrémités de la tige 6 peuvent, par ailleurs, avoir, d'une manière générale, une forme anguleuse intérieure, comme représentée sur la figure 7, ou extérieure, le contour de cette forme pouvant être quelconque, par exemple polygonal à n côtés, et approprié à celui de la clé permettant de faire tourner la tige 6 sur son axe.

## Revendications

1. Dispositif de fixation de tiges d'ostéosynthèse rachidienne sur des vis pédiculaires (1) du type comprenant une tête (4) en forme de diapason définissant deux branches parallèles en regard (4a, 4b) délimitant entre elles un logement (5) d'axe général perpendiculaire à l'axe de la vis, susceptible de recevoir une tige (6) à fixer, laquelle est bloquée dans son logement (5) par une vis de serrage (7) engagée entre lesdites branches (4a, 4b), caractérisé en ce qu'il comporte, en outre, enfilée sur la tige et interposée entre la vis de serrage et le fond dudit logement (5), une bague fendue (9) dont les faces externes opposées (10) dirigées vers ladite vis de serrage (7) et ledit fond sont convexes et reçues dans des surfaces concaves complémentaires (11, 12) ménagées sur l'extrémité de la vis de serrage (7) et le fond du logement de réception (5) de la tige (6), ladite bague (9) autorisant, avant serrage de la vis (7), un certain débattement angulaire de la tige (6) par rapport à l'axe de la vis (1).

2. Dispositif suivant la revendication 1, caractérisé en ce que lesdites surfaces convexe (10) et concaves (11,12) de la vis de serrage (7) et du fond du logement (5) de réception de la tige (6) sont sphériques.

3. Dispositif suivant la revendication 1 ou 2, caractérisé en ce que la vis de serrage (7) a une pointe conique tronquée dans sa partie centrale pour définir ladite surface concave (11).

4. Dispositif suivant l'une des revendications 1 à 3, caractérisé en ce que le fond du logement (5) de réception de la tige (6) est muni, au droit des entrées dudit logement et de part et d'autre de ladite surface concave (12), d'évidements (15) de dégagement.

5. Dispositif suivant l'une des revendications 1 à 4, caractérisé en ce que les branches (4a,4b) de la tête (4) de la vis et la vis de serrage (7) sont chapeautées par un capuchon (8) muni d'un trou central (18) d'accès à l'empreinte de commande de ladite vis de serrage.

6. Dispositif suivant la revendication 5, caractérisé en ce que ledit capuchon (8) est muni sur son bord d'échancrures (17) opposées assurant un libre débattement à la tige (6).

7. Dispositif suivant l'une des revendications 1 à 6, caractérisé en ce que la tige (6) est munie à chaque extrémité d'une empreinte (20), en creux ou en relief, permettant, à l'aide de clés de configuration appropriée, la rotation de la tige autour de son axe.

## Patentansprüche

1. Vorrichtung zum Befestigen von Rückgratosteosynthesestäben auf Stützschrauben (1) des Typs mit einem Kopf (4) in Form einer Stimmgabel, welche zwei Zinken (4a,4b) begrenzt, die zwischeneinander einen zu befestigenden Stab (6) aufnehmen, der in seiner Aufnahme (5) durch eine Spannschraube (7) blockiert wird, dadurch gekennzeichnet, daß sie ferner, aufgefädelt auf dem Stab (6) und eingefügt zwischen der Spannschraube (7) und dem Boden der Aufnehmung (5) einen geschlitzten Ring (9) umfaßt, dessen einander gegenüberliegende, gegen die Spannschraube (7) beziehungsweise den Boden gerichtete Außenseiten (10) konvex sind und in konkaven komplementären Oberflächen (11,12) aufgenommen sind, die auf dem Ende der Spannschraube (7) beziehungsweise dem Boden der Aufnahme (5) für den Stab (6) ausgebildet sind, wobei der Ring (9) vor dem Spannen der Schraube (7) eine bestimmte Winkelverlagerung des Stabes (6) relativ zur Achse der Schraube (1) ermöglicht.

2. Vorrichtung nach Anspruch 1, dadurch gekennzeichnet, daß die konvexen (10) und konkaven (11,12) Oberflächen der Spannschraube (7) und des Bodens der Aufnahme (5) für das Aufnehmen des Stabes (6) sphärisch sind.

3. Vorrichtung nach Anspruch 1 oder 2, dadurch gekennzeichnet, daß die Spannschraube (7) eine kegelstumpfförmige Spitze in ihrem Zentralabschnitt aufweist zum Definieren der konkaven Oberfläche (11).

4. Vorrichtung nach einem der Ansprüche 1 bis 3, dadurch gekennzeichnet, daß der Boden der Aufnahme (5) zum Aufnehmen des Stabes (6) an der Einlaßstelle der Aufnahme und beidseits der konkaven Oberfläche (12) mit Freiraum-Ausnehmungen (15) versehen ist.

5. Vorrichtung nach einem der Ansprüche 1 bis 4, dadurch gekennzeichnet, daß die Arme (4a,4b) des Kopfes (4) der Schraube und die Spannschraube (7) von einer Kappe (8) abgedeckt sind, die mit einem zentralen Zugangsloch (18) für den Betatigungseingriff der Spannschraube versehen ist.

6. Vorrichtung nach Anspruch 5, dadurch gekennzeichnet, daß die Kappe (8) auf ihrem Rand mit einander gegenüberliegenden Einschnitten (17) versehen ist, die ein freies Verkanten des Stabes (6) sicherstellen.

7. Vorrichtung nach einem der Ansprüche 1 bis 6, dadurch gekennzeichnet, daß der Stab (6) an jedem Ende mit einem Eingriff (20) in hohler oder Relief-Form versehen ist, der mit Hilfe eines Schlüssels entsprechender Konfiguration die Verdrehung des Stabes um seine Achse ermöglicht.

## Claims

1. Device for fixing spinal osteosynthesis rods on pedicular screws (1) of the type including a diapason
shaped head (4) defining two facing parallel branches (4a, 4b) defining together a housing (5) having an axis generally perpendicular to the screw axis , for receiving a rod (6) to be fixed, which is blocked in its housing (5) by a tightening screw (7) inserted between said branches (4a, 4b), characterized in that it further comprises, fitted onto the rod and interposed between the locking screw and the bottom of said housing (5), a split ring (9) whose opposing external faces (10) directed towards said tightening screw (7) and said bottom are convex and received in complementary concave surfaces (11, 12) provided on the end of the tightening screw (7) and the bottom of the housing (5) receiving the rod (6), said ring (9) authorizing a certain angular clearance of the rod (6) relative to the axis of the screw (1) prior to tightening of the screw (7).

2. Device according to claim 1, characterized in that said convex (10) and concave (11, 12) surfaces of the tightening screw (7) and the bottom of the housing (5) receiving the rod (6) are spherical.

3. Device according to claim 1 or 2, characterized in that the tightening screw (7) has a truncated conical apex in its central portion so as to define said concave surface (11).

4. Device according to any one of claims 1 to 3, characterized in that the bottom of the housing (5) receiving the rod (6) is provided, at the level of the inlets of said housing and on both sides of said concave surface (12), with clearance recesses (15).

5. Device according to any one of claims 1 to 4, characterized in that the branches (4a, 4b) of the head (4) of the screw and the tightening screw (7) are covered by a cap (8) provided with a central hole (18) for access to the recess for driving said tightening screw.

6. Device according to claim 5, characterized in that said cap (8) is provided on its edge with opposing recesses (17) providing free clearance to the rod (6).

7. Device according to any one of claims 1 to 6, characterized in that the rod (6) is provided at each end with a hollow or protruding pattern making it possible, with the aid of suitably sized wrenches, to rotate the rod around its axis.
